Europäisches Patentamt

European Patent Office

Office européen des brevets

(19)

(11) **EP 0 411 503 B1**

(12) # EUROPEAN PATENT SPECIFICATION

(45) Date of publication and mention
of the grant of the patent:
**15.12.1999 Bulletin 1999/50**

(51) Int Cl.[6]: **C07K 7/06**, C07K 7/08,
C07K 1/14

(21) Application number: **90114484.0**

(22) Date of filing: **27.07.1990**

(54) **Process for identifying and synthesizing binding sites of interacting proteins**

Verfahren zur Identifizierung und Herstellung von Bindungsstellen aufeinanderwirkender Proteine

Procédé pour l'identification et la synthèse des sites de liaison de protéines interagissants

(84) Designated Contracting States:
**AT BE CH DE DK ES FR GB GR IT LI LU NL SE**

(30) Priority: **31.07.1989 IT 2139689**
**25.06.1990 IT 2075590**

(43) Date of publication of application:
**06.02.1991 Bulletin 1991/06**

(73) Proprietor: **TECNOGEN**
**Società Consortile per azioni**
**81015 Piana di Monte Verna (Caserta) (IT)**

(72) Inventor: **Fassina, Giorgio**
**I-35128 Padova (IT)**

(74) Representative: **Gervasi, Gemma, Dr. et al**
**NOTARBARTOLO & GERVASI Srl,**
**Corso di Porta Vittoria, 9**
**20122 Milano (IT)**

(56) References cited:
**WO-A-86/05208**

- **PEPTIDES, CHEMISTRY AND BIOLOGY, PROCEEDINGS OF THE 10TH AMERICAN PEPTIDES SYMPOSIUM, St. Louis, 23rd - 28th May 1987, pages 354-362, ESCOM, Leiden, NL; I. CHAIKEN et al.: "Sequence simplification and randomization and the design of peptide recognition surfaces"**

- **7TH INTERNATIONAL SYMPOSIUM ON AFFINTY CHROMATOGRAPHY AND INTERFACIAL MACROMOLULAR INTERACTIONS, Oberammergau, 17th - 21st August 1987, BIOL. CHEM. HOPPE-SEYLER, vol. 368, no. 7, 1987, pages 737-738; I.M. CHAIKEN et al.: "Analytical affinity chromatography as a tool in the synthetic design of peptide and protein recognition surfaces"**
- **CHEMICAL ABSTRACTS, vol. 104, no. 21, 26th May 1986, page 235, abstract no. 181934s, Columbus, Ohio, US; J.E. BLALOCK et al.: "Binding of peptides that are specified by complementary RNAs"**
- **ADVANCES IN CHROMATOGRAPHY, vol. 27, 1987, pages 247-298, Marcel Dekker, Inc., New York, US; G. FASSINA et al.: "Analytical high-performance affinity chromatography"**
- **TECHNIQUES IN PROTEIN CHEMISTRY, 2ND ANNUAL MEETING, San Diego, CA, 13th - 17th August 1988, pages 430-438, Academic Press; J.G. OMICHINSKI et al.: "Computer assisted design of recognition peptides"**
- **JOURNAL OF BIOLOGICAL CHEMISTRY, vol. 264, no. 19, 5th July 1989, pages 11252-11257, American Society for Biochemistry and Molecular Biology, US; G. FASSINA et al.: "Recognition properties of peptides hydropathically complementary to residues 356-375 of the c-raf protein"**
- **CELL, vol. 55, no. 3, 4th November 1988, pages 497-504, Cell Press; F. MAZEROLLES et al.: "Immunosuppressive properties of synthetic peptides derived from CD4 and HLA-DR antigens"**

- CHEMICAL ABSTRACTS, vol. 113, no. 1, 2nd July 1990, page 302, abstract no. 3014v, Columbus, Ohio, US; & US-A-322 266 (UNITED STATES DEPT. OF HEALTH AND HUMAN SERVICES) 15-08-1989

**Description**

BACKGROUND OF THE INVENTION

The determination of the active sites of interacting proteins is of enormous importance since it offers the possibility of synthesizing and possibly of suitably modifying amino acid sequences capable of recognizing a certain protein and thus of interacting with it. Many works were carried out to predetermine these interacting structures in order to enable their easy reproduction. For example the relationship existing between the hydropathy of amino acids and the amino acids encoded by codons complementary with the formers is known (J.E. Blalock and E. M. Smith, BBCR, 121:203, 1984).

It is also known that peptides expressed by complementary DNAs are capable of interacting with one another (K. L. Bost et al. PNAS, 82:1372, 1985).

In the present text the expression "average hydropathy flowing along the protein chain" means calculating by sequential method the arithmetic mean of hydropathic values of each aminoacid along the chain in groups of 3,5,7,9,11 residues.

On the basis of these knowledges the hormon-receptor binding sites were argued to derive form the transcription of complementary RNA strands (K. L. Bost et al. PNAS, 82:1372, 1985). This hypothesis was confirmed by the identification of the interaction site between IL-2 and its receptor.

To apply this identification method for the contact domain between interacting proteins, the knowledge of the sequence of the nucleic acid encoding the interacting proteins is, as said, necessary. The research of the nucleotide complementary within the RNA sequence is however cumbersome and often complicated by the presence of stop codons; moreover such a method was found to be ineffective for identifying the sites of nucleotide complementarity between the P-substance and its receptor (A: Goldstein and D. L. Brutlag, PNAS, 86:42, 1989). The development of a method more flexible and reliable than the aforementioned method, which enables the more precise determination of the protein binding sites, is therefore particularly important.

The main strength regulating the interaction between peptides expressed by complementary RNAs was recently found to be the hydropathic complementary determined as average hydropathy flowing along the protein chain (G. Fassina et al. J. Biol. Chem. 264:11252, 1889; G. Fassina et al. Methods in Protein Sequence Analysis, 431:439, 1989).

G. Fassina et al. J. Biol. Chem. 264:11252, 1989, describes the design of a peptide complementary to the raf-(356-375) peptide by means of a computer program which assigns the appropriate hydropathy value based on the Kyte and Doodlittle hydropathy scale to each residue of the target peptide, then it selects possible hydropathically complementary amino acids for each target peptide residue, using a moving segment approach that continuosly determines the average hydropathy within a segment of a predetermined length as it advances through the sequence of the target peptide. Similarly, for each selected segment, complementary sequences that maximize the degree of average hydropathic complementarity are chosen.

As pointed out hereinafter in the present application, this document refers to the MAHS method, which is also described in Techniques in protein Chemistry, 2nd Annual Meeting, San Diego, CA, 13th-17th August 1988, pages 430-438, Academic Presss; J. G. Omichinski et al.: "Computer assisted design of recognition peptides".

The above finding enabled the development of the process of synthesis which is the object of the present invention and which allows the solution of the above drawbacks is that:

    a) it does not require for the analysis knowledge about the nucleotide sequence;
    b) the determination of the domains of hydropathic complementarity is easier and quicker than the research of complementary nucleotides since it does not require particular algorithm;
    c) the contact sites are always located within the interacting protein sequence.

It is therefore possible to foresee the sites in which two given proteins will interact merely on the basis of their amino acid sequence. Said sites may thereafter be reproduced by chemical synthesis and be used alone or conjugated to other molecules. Moreover the sites identified according to the present method may be modified either by chemical reaction or by direct mutagenesis of the same sites in the protein under consideration.

Furthermore, the new technique indicated as AMINOMAT is characterized by a hydropathic complementarity score lower than that obtained by the MAHS method.

SUMMARY

The present invention is directed to the synthesis of a complementary amino acid sequence having high affinity and selectivity in recognizing a target peptide or protein sequence, which more particulalry is of natural origin and avaialable, and is identified as the binding site between two proteins, by means of a process which comprises steps a) to h) hereinafter reported in the present text, and is characterized in that it uses Table 2, hereinbelow disclosed. According to a particular embodiment of the present invention, the binding site between two proteins both of natural origin is identified by means of a process comprising steps 1) to 6) as hereinafter described in the present text, which uses the hydropathy scale according to Table 1 hereinafter reported.

Further objects of the present invention are represented by the new peptides $NH_2$-APPISGOIRSS-COOH and $NH_2$-GPSKLNDRADS-COOH, HLLFPIIIAASL and RKFLAGLRARRLKR, the preparation thereof is hereinafter illustrated, and by an affinity column for purifying proteins and peptides consisting of the new peptides hereinabove mentioned, binded on a suitable solid support.

DETAILED DESCRIPTION OF THE INVENTION

The process of synthesis of peptides identified as the active binding sites between two proteins comprises the following steps:

1) determination of the amino acid sequence of the two interacting proteins at issue (Pa) and (Pb);
2) determination of the hydropathic value for each element of the amino acid sequence of both protein by means of the hydropathic value scale (TABLE 1) described below;
3) sum of the hydropathic values for the two proteins in such a way to obtain the everage value of hydropathy flowing along the protein chain, said $a_i$ and $b_i$ respectively for the former and the latter protein, according to the formulae:

$$a_i = \Sigma \, h_k \, (P_k)/r \qquad b_i = \Sigma \, h_k \, (P_k)/r$$

where $i = (i+s)$ and $k = (i-s)$ and where r is an integer defined as the run length $(r<n)$ and s is $(r-1)/2$ per each residue i of the former and latter sequence;
4) calculation of the complementary hydropathic score defined according to the formula:

$$\theta = [\Sigma \, (a_i + b_i)^2/L]^{1/2}$$

by a given length of search L expressed as number of amino acids, where L is lower than the total number of amino acids comprised in the sequences Pa and Pb, for all the alternative fragments of length L comprised in the first sequence Pa:

$$P_1 \text{-} P_L \; P_2 \text{-} P_{L+1} \; ..... P_{(n-L+1)} \text{-} P_n$$

with all the possible fragments of the same L length from the latter sequence Pb.
In particular pondered scores defined on the basis of the formula:

$$W\theta_j = \theta_j/[\,(\Sigma \, abs \, (a_j) + abs \, (b_j))/2*L]*10$$

are preferred.
5) Selection of at least a pair of sequences of length L from the interacting couple Pa and Pb, exhibiting the lowest score, which are identified as contact sequences;
6) synthesis according to known techniques of the sequence under consideration.

According to a peculiar embodiment of the present invention, the two interacting proteins for which the amino acid sequence is elucidated, are both of natural origin and available.

According to a further embodiment of the invention, one of the two proteins only is of natural origin and available (target protein) while the second is purposely synthesized using the process of synthesis according to the present invention.

In this specific embodiment of the invention, the process is characterized as follows:

a) the amino acid sequence Pi of the target peptide or protein is elucidated:

$$Pi = P_1 - P_2 - P_3 - P_4 \ldots P_n;$$

b) the hydropathic value **h** to be attributed to each residue of the sequence P (target) is deduced by means of a predetermined scale (table 2) described below:

$$h_1 - h_2 - h_3 - h_4 \ldots h_n;$$

c) a set of complementary amino acids Q is attributed to each residue of the sequence P:

$$P_1 - \rightarrow h_1 - \rightarrow (Q_{1,1} - Q_{1,2} - Q_{1,3} \ldots Q_{1,m})$$
$$P_2 - \rightarrow h_2 - \rightarrow (Q_{2,1} - Q_{2,2} - Q_{2,3} \ldots Q_{2,m})$$
$$P_n - \rightarrow h_n - \rightarrow (Q_{n,1} - Q_{n,2} - Q_{n,3} \ldots Q_{n,m})$$

d) the hydropathic values of the target sequence are summed in order to obtain the average value of hydropathy flowing along the sequence according to the formula:

$$a_i = \Sigma_k h_r (P_k)/r$$

where $a_i$ is the average value of flowing hydropathy, h is the hydropathic value, P is the peptide sequence, **k** is the constant comprised within (i-s) and (i+s), **i** is higher than s and lower than or equal to (n-s), **s** is (r-1)/2, with **r** odd number equal to or lower than **n** and **n** is the number of amino acids comprised in the sequence under consideration;

e) the hydropathic values of the series of the hydropathically complementary amino acids are summed in such a way to obtain the average values of flowing hydropathy for each member of the complementary series according to the formula:

$$b_{j,i} = \Sigma_k h'_{j,k}(P_{j,k})/r$$

where $b_{j,i}$ is the average value of the flowing hydropathy, **k** is comprised between (i-s) and (i+s), **i** is higher than s and lower than or equal to (n-s), **j** is comprised between 1 and the total number of sequences of the complementary series, s is (r-1)/2, **r** is an odd number lower than or equal to **n, n** is the total number of amino acids comprised in the complementary series;

f) the score of hydropathic complementarity θ is calculated and defined as:

$$\theta = [\Sigma(a_{i,k} + b_{j,i,k})^2 / (n-2S)]^{1/2}$$

where **k** is comprised between (s-1) and (s+1), **n** and **s** are defined as above;

g) the complementary sequence exhibiting the lowest complementarity score θ is selected and identified as that having high affinity and selectivity in recognizing the target sequence;

h) the complementary aminoacid sequence identified by the lowest complementarity score is synthesized according to known techniques.

## LIST OF DRAWINGS

**FIG. 1** The two lines on the graphic represent the complementary hydropathic values of the sequences 51-57 and 57-51 (antiparallel sequence from C terminal to N terminal end) of the BNP 1 (as per Table 1);

**FIG. 2** the two lines on the graphic represent the values of complementary hydropathy of the sequences 429-440 of the protein HIV-1 gp120 and 61-51 of the protein CD4 (Table 1);

**FIG.3** shows the elution chromatogram of the CD4 peptide on a column containing the HIV-1 gp 120 peptide:

**FIG.4** the lines on the graphic represent the hydropathic values of the sequence HIV-1 gp120 (429-439) following the methods MAHS and AMINOMAT;

**FIG.5** shows the chromatograms of the elution of the equal amounts of complementary peptides on the affinity column;

**FIG.6** shows the elution diagrams of different amounts of complementary peptides, obtained by calculating the delay volume V for each elution and the dissociation constants by extrapolating to zero the concentrations of peptide;

**FIG.7** shows the elution of IL-1β[205-216] by 0.1 M HAc at pH 2.5;

**FIG.8** shows the elution of retained material by 0.1 M HAc;

**FIG.9** shows RP-HPLC analysis to confirm the presence of IL-1β[205-216] and to evaluate the extent of purification of a crude peptides mixture;

**FIG.10a** shows the HPLAC elution profile of 20 μg of IL-1β STD on the affinity column;

**FIG.10b** shows the evaluation of purity through HPLC;

**FIG.10c** shows the evaluation of purity through HPEC;

**FIG.11** shows the elution profiles of IL-1β[205-216] and of IL-1β on an affinity column derivatized with TRIS;

**FIG.12** shows the hydropathy profiles of the complementary peptides ET[16-32] and CGET;

**FIG.13** shows the elution profile of the peptide [16-32] by means of HPLAC derivatized with TRIS;

**FIG. 13a** shows the RP-HPLC elution profile of the peptide ET[ 16-32];

**FIG. 14** shows the HPLAC elution profile of the peptides ET[16-32] and Big ET[1-38] from crude complex mixtures;

**FIG. 15** shows the RP-HPLC elution profiles of the peptides ET[16-32] and Big ET[1-38] from crude complex mixtures;

**FIG.16** shows the chromatograms of the affinity column suitable for quantifying ET[1-38] in the range 2 to 40 μg;

**FIG. 17** shows the calibration curve of the column of Fig. 16;

**FIG. 18** shows the degradation rate of the peptide ET[16-32] by enzyme chymotrypsin;

**FIG. 19** shows the degradation rate of the complex of the complementary couple of the peptides ET[16-32] and CGET by enzyme chymotrypsin;

**FIG. 20** shows the degradation rates of the peptide ET[16-32] and of the complex of the complementary couple of the peptides ET[16-32] and CGET by enzyme chymotrypsin.

EXPERIMENTAL PART

The amino acid sequences of the protein HIV-1 gp120 and of its receptor CD4 are known in the literature (B. Starcich et al., Cell, 45:637, 1986; P. J. Maddon et al. Cell, 42:93, 1985). The sequence of the first extracellular ring of the receptor CD4 comprising the binding region for the HIV-1 gp120 (N. E. Richardson et al., Proc. Natl. Acc. Sci. 85:6102, 1988) and the residues comprised in the region 101-504 of the HIV-1 gp120 were analysed in order to determine hydropathically complementary sequences.

In this sequences system, the analysis has been carried out by using the hydropathy scale herein disclosed as reference:

TABLE 1:

| AminoAcid | Value | Amino-Acid | Value |
|---|---|---|---|
| Ala (A) | 2.8 | Leu (L) | 4.8 |
| Arg (R) | -3.5 | Lys (K) | -2.9 |
| Asn (N) | -2.5 | Met (M) | 2.9 |
| Asp (D) | -2.5 | Phe (F) | 3.8 |
| Cys (C) | 3.5 | Pro (P) | -0.6 |
| Gln (Q) | -2.5 | Ser (S) | 0.2 |
| Glu (E) | -2.5 | Thr (T) | 0.3 |
| Gly (G) | 0.6 | Trp (W) | 0.1 |
| His (H) | -2.2 | Tyr (Y) | -0.3 |
| Ile (I) | 5.5 | Val (V) | 5.2 |

This scale was derived from the analysis of the hydropathic profile of the Bovine Neurophysine I (BNP I), determined according to Kyte and Doolittle (J. Mol. Biol. 157:105, 1982). BNP I is capable of dimerizing and the interaction sites are localized near by the unique tyrosine residue at the position 49, as proved by the studies of protonic nuclear magnetic resonance (Peyton et al, Biochemistry, 25:6579, 1986). The BNP I sequence 48-57, i.e. NYLPSPCQSGQ is complementary to itself, if ranged in an antiparallel sense.

When the values given by the above scale are graphically reproduced, the deviation of one hydropathy unit of the same values with regard to the reference zero value (corresponding to the complementarity) is observed; to obviate this one hydropathy unit was added to the hydropathic values of all twenty amino acids obtaining in such a way the table of values above reported.

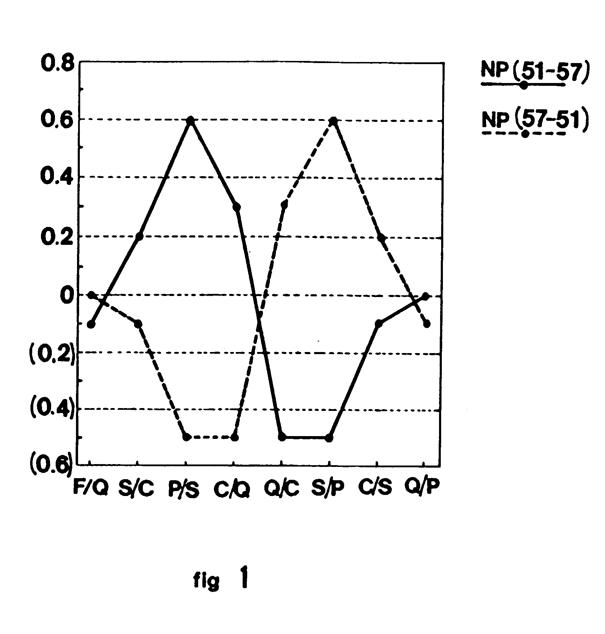

By using this modified scale, the hydropathic profiles of the BNP I sequences 48-57 and 57-48 (antiparallel sequence from C terminal to N terminal end) are hydropathically complementary with reference to the zero value of the scale (Fig. 1).

By making use of said scale the sequences of the HIV-1 gp120 (in the usual direction N terminal to C terminal end) and that of the CD4 (in reverse direction i.e. from C terminal to N terminal end) were analysed.

The ten pairs of sequences characterized by the lowest scores are reported hereinafter, both for the pondered (a) and not pondered (b) scores:

```
MOVING AVERAGE        :      7
---------------------------------------------------------------
1 : MYAPPISGQIR
2 : RRSDARDNLKS
Begin Chain 1: 327 - Begin chain 2:  26 - leng.:  11
Score:    2.89200
---------------------------------------------------------------
1 : YAPPISGQIRC
2 : RSDARDNLKSP
Begin Chain 1: 328 - Begin chain 2:  27 - leng.:  11
Score:    2.89200
---------------------------------------------------------------
1 : PEIVTHSFNCG
2 : LSRRSDARDNL
Begin Chain 1: 267 - Begin chain 2:  24 - leng.:  11
Score:    2.93877
---------------------------------------------------------------
1 : DPEIVTHSFNC
2 : WLSRRSDARDN
Begin Chain 1: 266 - Begin chain 2:  23 - leng.:  11
Score:    3.07482
---------------------------------------------------------------
1 : MRQAHCNISRA
2 : WHFQISKKQSA
Begin Chain 1: 224 - Begin chain 2:  57 - leng.:  11
Score:    3.33030
---------------------------------------------------------------
1 : LFNSTWSTKGS
2 : RDNLKSPGKTL
Begin Chain 1: 288 - Begin chain 2:  31 - leng.:  11
Score:    3.41121
---------------------------------------------------------------
1 : EIVTHSFNCGG
2 : SRRSDARDNLK
Begin Chain 1: 268 - Begin chain 2:  25 - leng.:  11
Score:    3.45096
---------------------------------------------------------------
1 : SEIFRPGGGDM
2 : HFQISKKQSAT
Begin Chain 1: 358 - Begin chain 2:  58 - leng.:  11
Score:    3.47720
---------------------------------------------------------------
1 : EIFRPGGGDMR
2 : FQISKKQSATC
Begin Chain 1: 359 - Begin chain 2:  59 - leng.:  11
Score:    3.50025
---------------------------------------------------------------
1 : STKGSHHTEGS
2 : PGKTLFSGQHG
Begin Chain 1: 294 - Begin chain 2:  37 - leng.:  11
Score:    3.59292
```

B) Moving average :    7
------------------------------------------------------------

```
1 : YAPPISGQIRC
2 : RSDARDNLKSP
Begin Chain 1: 328 - Begin chain 2:   27 - leng.:   11
Score:    3.61499
```
------------------------------------------------------------

```
1 : MYAPPISGQIR
2 : RRSDARDNLKS
Begin Chain 1: 327 - Begin chain 2:   26 - leng.:   11
Score:    3.61499
```
------------------------------------------------------------

```
1 : PEIVTHSFNCG
2 : LSRRSDARDNL
Begin Chain 1: 267 - Begin chain 2:   24 - leng.:   11
Score:    3.67346
```
------------------------------------------------------------

```
1 : DPEIVTHSFNC
2 : WLSRRSDARDN
Begin Chain 1: 266 - Begin chain 2:   23 - leng.:   11
Score:    3.84353
```
------------------------------------------------------------

```
1 : CTRPNNNTRKK
2 : IKLNKIILPFN
Begin Chain 1: 194 - Begin chain 2:    9 - leng.:   11
Score:    3.96481
```
------------------------------------------------------------

```
1 : GKAMYAPPISG
2 : WLSRRSDARDN
Begin Chain 1: 324 - Begin chain 2:   23 - leng.:   11
Score:    4.29025
```
------------------------------------------------------------

```
1 : EIVTHSFNCGG
2 : SRRSDARDNLK
Begin Chain 1: 268 - Begin chain 2:   25 - leng.:   11
Score:    4.31369
```
------------------------------------------------------------

```
1 : AMYAPPISGQI
2 : SRRSDARDNLK
Begin Chain 1: 326 - Begin chain 2:   25 - leng.:   11
Score:    4.39365
```
------------------------------------------------------------

```
1 : KAMYAPPISGQ
2 : LSRRSDARDNL
Begin Chain 1: 325 - Begin chain 2:   24 - leng.:   11
Score:    4.39365
```
------------------------------------------------------------

```
1 : NCTRPNNNTRK
2 : EIKLNKIILPF
Begin Chain 1: 193 - Begin chain 2:    8 - leng.:   11
Score:    4.43813
```
------------------------------------------------------------

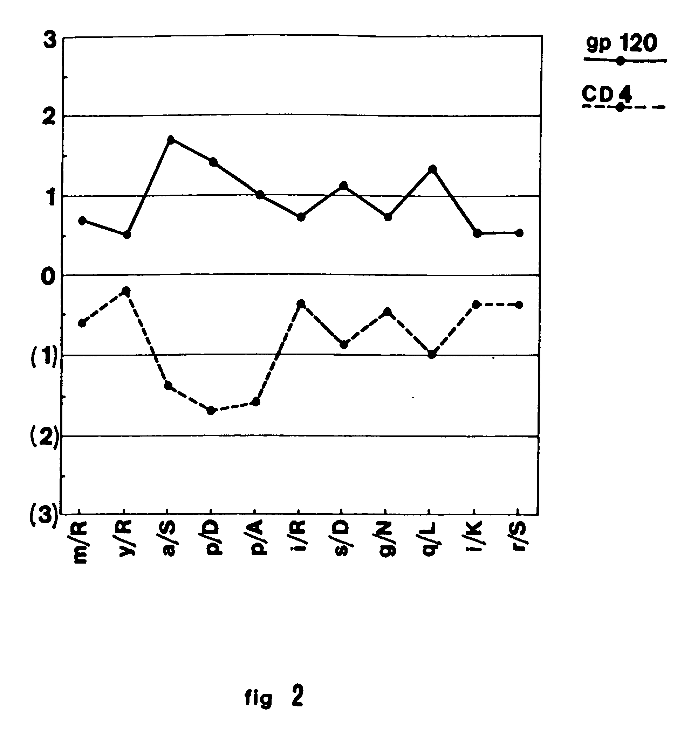

According to both methods the complementary sequences 429-440 of the proteins HIV-1 gp120 and 61-51 of the CD4 were identified as being the contact sequences on the basis of their lowest score. It is interesting to note that the sequence HIV-1 gp120 identified according to the present method is located within the region 406-449 of the HIV-1 gp120 molecule, previously pointed out as an important domain for the interaction with the receptor (L. A. Lasky et al., Cell, 50:975, 1987). The hydropathic profiles of the two contact sequences are reported in fig. 2. The average values of flowing hydropathy have been obtained according to the hydropathy scale referred to above.

Two peptides comprised in the complementary region, i.e. NH2-APPISGOIRSS-COOH for the HIV-1 gp120 protein and NH2-GPSKLNDRADSCOOH for the receptor CD4 were produced by chemical synthesis and purified to chromatographic homogeneity by HPLC in reverse phase. The chemical nature of the peptides was confirmed by amino acid analysis.

The existence of an interaction ability among complementary peptides was verified by affinity HPLC (G. Fassina and I. M. Chaiken, Advances in Chromatogr. 27:250, 1987).

The peptide HIV-1 gp120 was immobilized on a solid support (Eupergit$^R$ C 30n, 800 μmoles/g load capability by controlled density) according to the conditions reported in literature (G. Fassina et al., J. Chromatogr. 376:87, 1986) and an HPLC glass column (80∗ 6.6 mm ID) was loaded with the peptide derivatized support to prepare an affinity column. The total amount of immobilized peptide in the column is 260 μg, as checked from the amino acid analysis of the support. Small amounts (1-10 μg) of CD4 peptide were eluted isochrathically on the column equilibrated by a flow rate of 1.0 ml/min with 0.1 M $NH_4Ac$, pH 5.7.

The eluted solution was analysed by monitoring the optic absorbance.

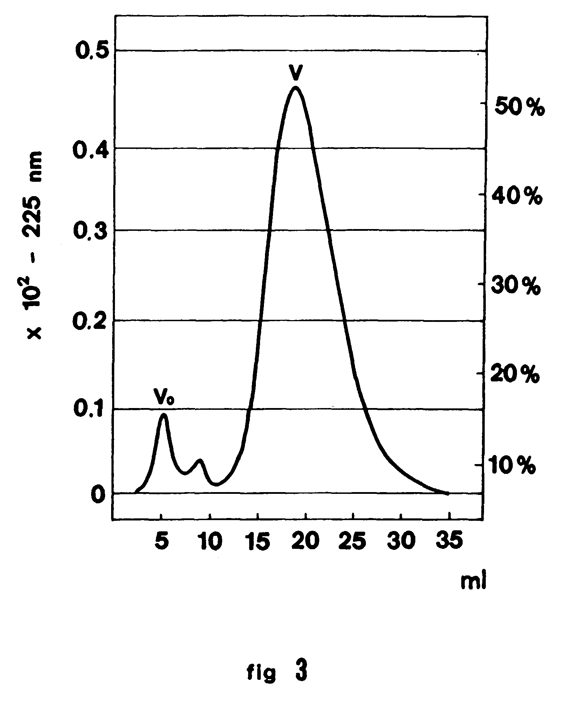

A typical chromatogram for the elution on a column containing the HIV-1 gp120 peptide of the CD4 peptide is reported in fig. 3.

The extension of the delay on column, which indicates the interaction between the peptides, was correlated to the dissociation constant Km/p of the binding according to the equation:

$$1/(V-V_o) = K_{m/p}/M_T + [P]_T/M_T$$

where V is the elution volume of the eluted peptide, $V_o$ is void volume of the column, $M_T$ is the total immobilized peptide, $[P]_T$ is the eluted peptide, and $K_{m/p}$ is the dissociation constant of the binding.

Many elutions were carried out with varying amounts of peptide CD4 and for extrapolation at concentration zero of peptide, a dissociation constant of $8*10^{-5}$ M for the peptide interaction was obtained.

Competitive binding assays where 1 mM of HIV-1 gp120 was added to the buffer proved that the interaction is peptide-specific. In fact at the aforementioned conditions the binding is completely inhibited. Moreover other peptides do not interact with HIV-1 gp120 at the above experimental conditions. The confirmation of the validity of the embodiment of the invention wherein one protein only is of natural origin and available (target peptide) while the second is purposely synthesized was obtained preparing peptides complementary to the site 429-439 of the HIV-1 gp120 (APPISGQIRCS). The complementary peptides to the HIV-1 gp120 429-439 site were obtained according to the present process, calculating the average flowing value on seven residues and using both the scale of Table 1 for identifying and select its complementary natural peptide and the scale of Table 2 reported below for synthesizing one complementary peptide thereof.

TABLE 2

| AminoAcid | Hydropathic value | Inverses |
|---|---|---|
| Ala (A) | 1.8 | P S W Y |
| Arg (R) | - 4.5 | I L V |
| Asn (N) | - 3.5 | C I L F V |
| Asp (D) | - 3.5 | C I L F V |
| Cys (C) | 2.5 | N D Q E H P |
| Gln (Q) | - 3.5 | C I L F V |
| Glu (E) | - 3.5 | C I L F V |
| Gly (G) | - 0.4 | G |
| His (H) | - 3.2 | C L F V |
| Ile (I) | 4.5 | R N D Q E K |
| Leu (L) | 3.8 | R N D Q E H K |
| Lys (K) | - 3.9 | I L V |

TABLE 2   (continued)

| AminoAcid | Hydropathic value | Inverses |
|---|---|---|
| Met (M) | 1.9 | P W Y |
| Phe (F) | 2.8 | N D Q E H |
| Pro (P) | - 1.6 | A C M |
| Ser (S) | - 0.8 | A |
| Thr (T) | - 0.7 | A G |
| Trp (W) | - 0.9 | A M |
| Tyr (Y) | - 1.3 | A M |
| Val (V) | 4.2 | R N D Q E H K |

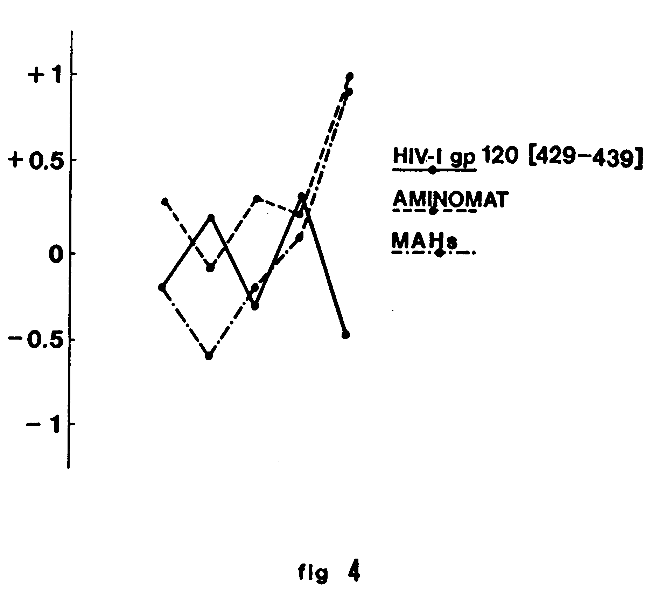

The sequences obtained with the corresponding hydropathic complementarity scores and hydropathy profiles are illustrated below in fig. 4.

The complementary sequence obtained according to the method AMINOMAT, name which indicates the new technique (while the name MAHS indicates the technique of the literature) is characterized by a hydropathic complementarity score lower than the score obtained by the MAHS method described in G. Fassina et al., J. Biol. Chem. 264: 11252-11257, 1989 and in G. Fassina et al. Methods in Protein Sequence Analysis 431-439, 1989.

In agreement with said previous studies, the lower the complementarity scores, the higher is the tendency of the complementary peptides to associate.

In order to experimentally evaluate the association ability among complementary peptides, the HIV-1 gp120 (429-439) peptide and the peptides deduced with the AMINOMAT and MAHS methods were produced by solid phase synthesis according to the method Fmoc, they were purified by HPLC and their correct chemical nature was confirmed by mean of amino acid analysis. The experimental evaluation of the association ability between complementary peptides was carried out by analytical affinity chromatography. The HIV-1 gp120 (429-439) peptide was immobilized on a solid support (Eupergit$^R$ C30 n) preactivated with epoxy-groups at the conditions referred to in literature. A glass HPLV column (80*-66 mm I.D.) was loaded with the peptide derivatized support to obtain an affinity column. The total amount of immobilized peptide on the column turned up to be 260 $\mu$g, as determined by amino acid analysis of the derivatized support. Small amounts (1-10 $\mu$g) of complementary peptide were eluted isocrathically on the column equilibrated by a flow rate of 1.0 ml/min with 0.1 M NaH$_2$ PO$_4$, pH 7.0.

The eluted solution was analysed by monitoring the optic absorbance.

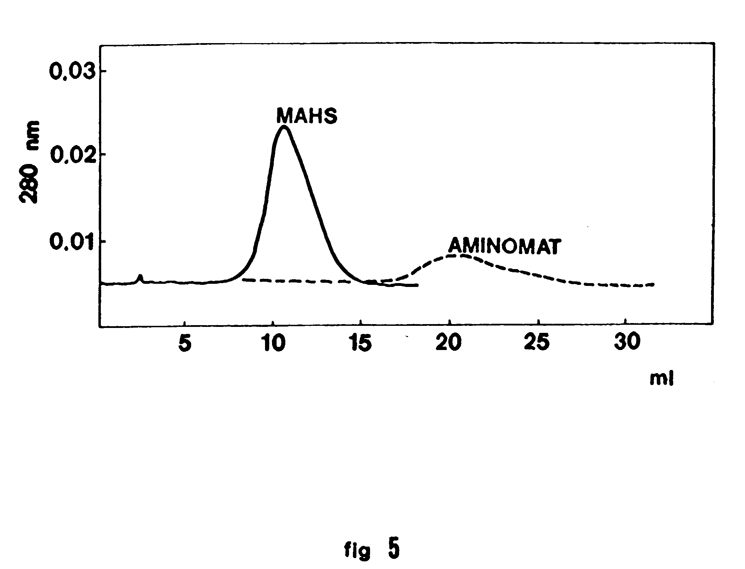

Some chromatograms relating to the elution of the same amount of complementary peptides on the affinity column are reported hereinafter in fig. 5.

The extension of the elution delay (elution volume) on column, which indicates the extension of the interaction, was correlated to the dissociation constant Km/p of the binding according to the equation:

$$1/(V-V_o) = K_{m/p}/M_T + [P]_T/M_T$$

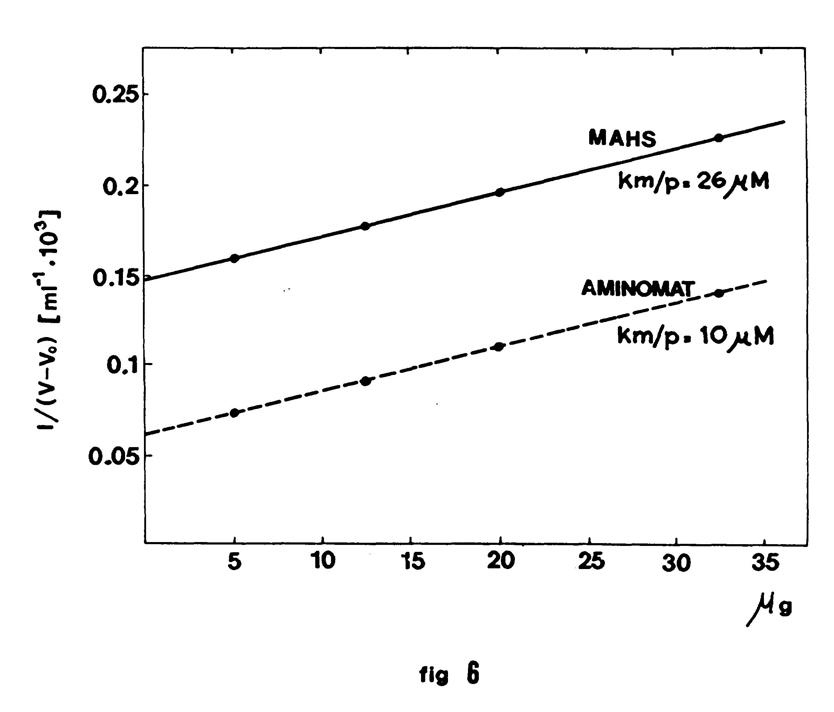

where V is the elution volume of the eluted peptide, $V_o$ is void volume of the column, $M_T$ is the total amount of immobilized HIV-1 gp120 (429-439) peptide, $[P]_T$ is the total amount of the eluted peptide, and $K_{m/p}$ is the dissociation constant of the interaction. Elutions with different amounts of complementary peptides were effected calculating for each elution the delay volume V and, for extrapolation to concentration zero of peptide, the dissociations constants were determined. The corresponding diagrams are reported in fig. 6.

The extrapolated values of dissociation constant show a higher affinity of the peptide produced according to the AMINOMAT method with regard to the complementary peptide obtained with the MAHS method. Competitive binding assays where the HIV-1 gp120 (429-439) peptide was added at the concentration 1mM to the elution buffer proved that the interaction is peptide specific. In fact the binding is completely inhibited at the aforementioned conditions.

At the same used elution conditions, several control peptides are not delayed by the column, showing the absence of specific bindings. Moreover the complementary peptides derived from the AMINOMAT and MAHS methods are not delayed by affinity columns of same size but derivatized with other molecules. In order to further evaluate the features of specificity and selectivity of the peptides obtained by the AMINOMAT method, a complementary peptide directed to the site [205-216] of the human interleukin 1$\beta$ [KQYPKKKMEKRF] and located on a loop exposed to the solvent and likely accessible for the binding with other molecules was designed.

By means of the present method and calculating the flowing average on 9 residues, the sequence HLLFPIIIAASL [CGIL] was obtained, which was characterized by a hydropathic complementary score of 0.01439. The peptides IL-1$\beta$ [205-216] and [CGIL] were synthesized by solid phase synthesis with the Fmoc method, purified to homogeneity on RP-HPLC and their correct chemical nature was confirmed by means of amino acid analysis. To verify the recognition ability of the complementary peptides of the pair, an affinity columns was prepared by immobilizing the [CGIL] on the support Eupergit [R] C30 n at known conditions.

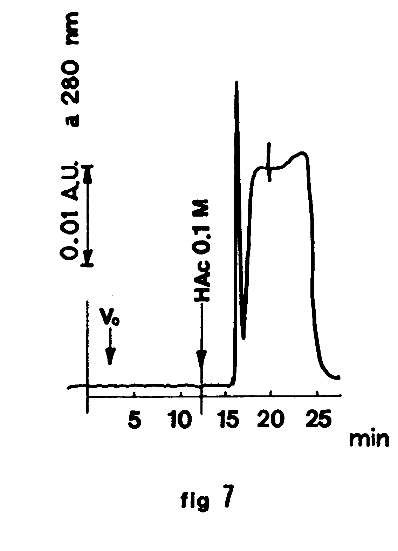

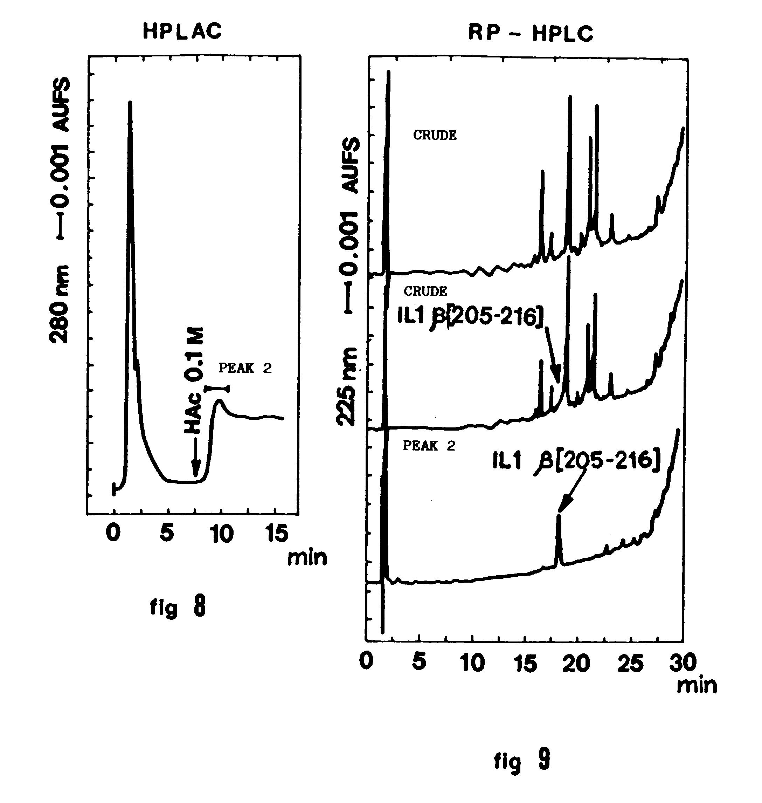

The total amount of immobilized [CGIL] was of 500 μmoles, determined by amino acid analysis of pondered amounts of derivatized support. The recognition of the complementary peptides of the pair, using 0.1 M TRIS pH 7.0 as equilibration buffer, was strong enough to need 0.1 M HAc pH 2.5 for eluting the IL-1β [205-216]. (FIG. 7). To show the selectivity of the binding between the complementary peptides of the pair, a mixture of several peptides (total concentration 1 mg/ml) added with the peptide IL-1β [205-216] at the concentration 1 μg/ml, was prepared. The amount corresponding to 20 ml of the peptide mixture comprising the IL- 1β [205-216] was injected into the affinity column equilibrated with 0.1 M TRIS pH 7.0 at a flow rate of 1.0 ml/min. After the complete elution of the nonretained material, the liquid phase was replaced by 0.1 M HAc for eluting the retained material. (Fig 8).

The 0.1 M HAc eluted fraction was analysed by RP-HPLC to confirm the presence of IL-1β [205-216] and to evaluate the extent of the purification. (Fig. 9).

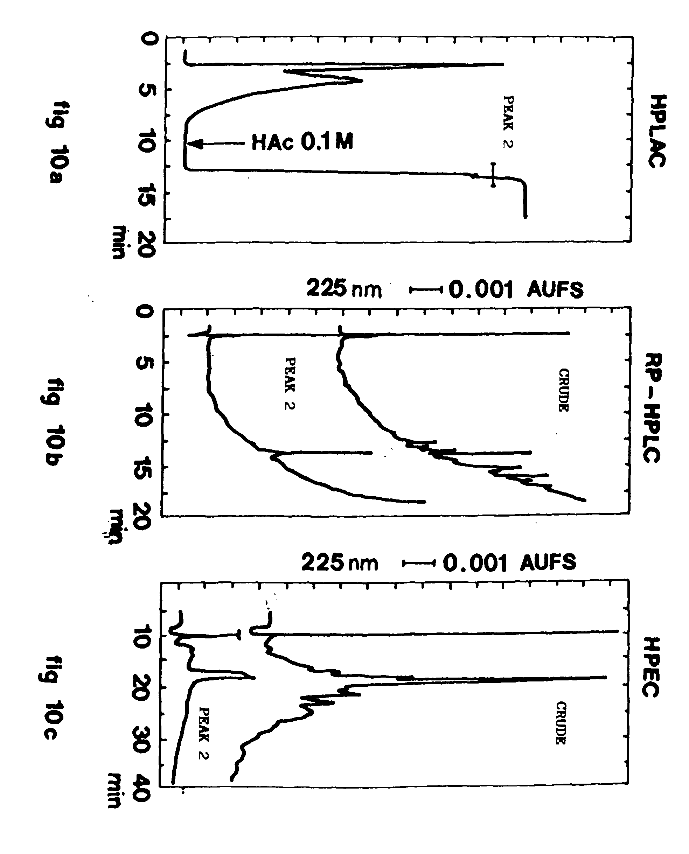

The ability of the immobilized complementary peptide [CGIL] to recognize the protein Interleukin 1β (365 residues) was also investigated. The elution profile of 20 μg of a crude mixture containing IL-1β recombinant on the affinity column and HPLC and HPEC determinations of purity thereof are reported in figures 9, 10a, 10b and 10c.

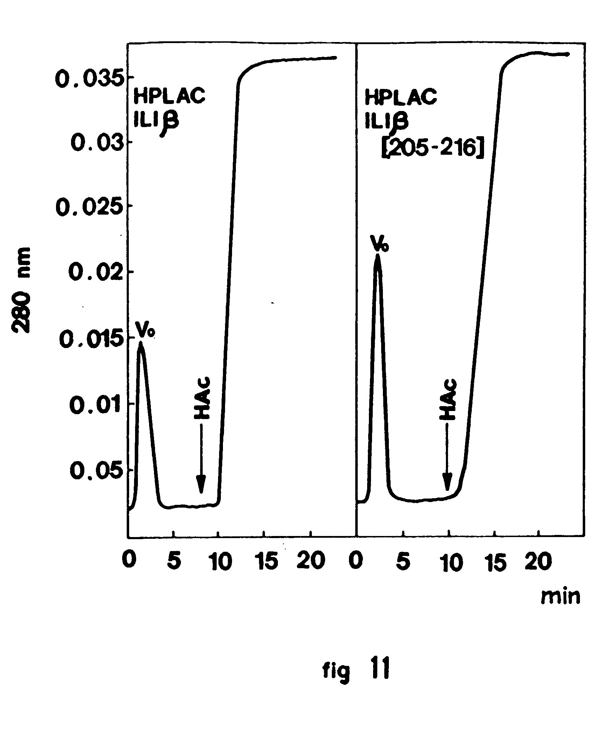

In this case too the recognition is strong enough to need the replacement of the eluent solution by 0.1 M HAc to elute the protein. The absence of non-specific bindings with the support was proved by eluting with TRIS buffer IL-1β [205-216] and IL-1β from an affinity column of the same size of the previously used but differently derivatized (fig. 11). At the aforementioned conditions neither the peptide nor the protein are delayed or retained on the column free of the complementary peptide [CGIL].

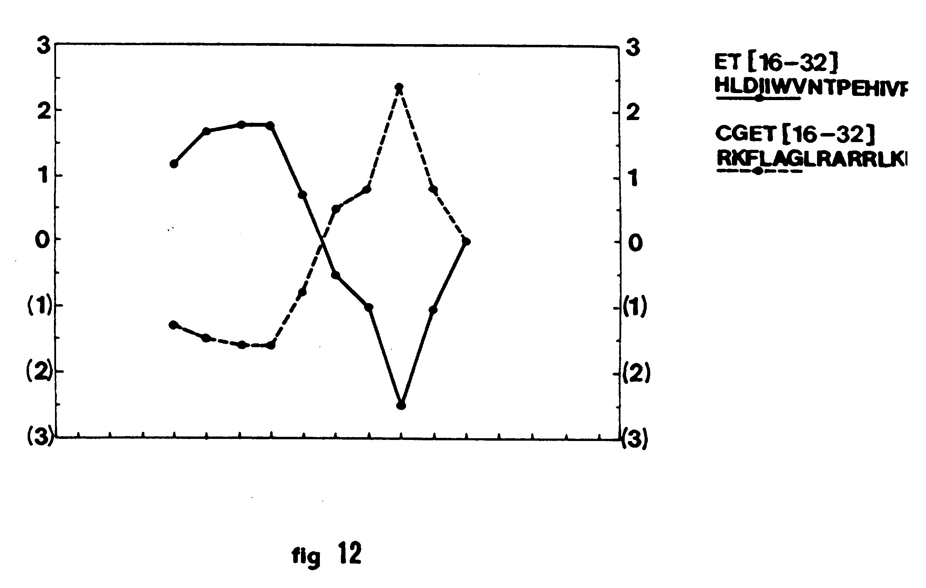

The method described in the present invention was also applied for designing a recognition peptide directed to the site 16-32 of Big Endotheline [1-38]. By using 9 residues for calculating the flowing average, the sequence obtained with the AMINOMAT method was RKFLAGLRARRLKR with a hydropathic complementarity score of 0.0357. The corresponding hydropathy profiles of the complementary peptides are reported in fig. 12.

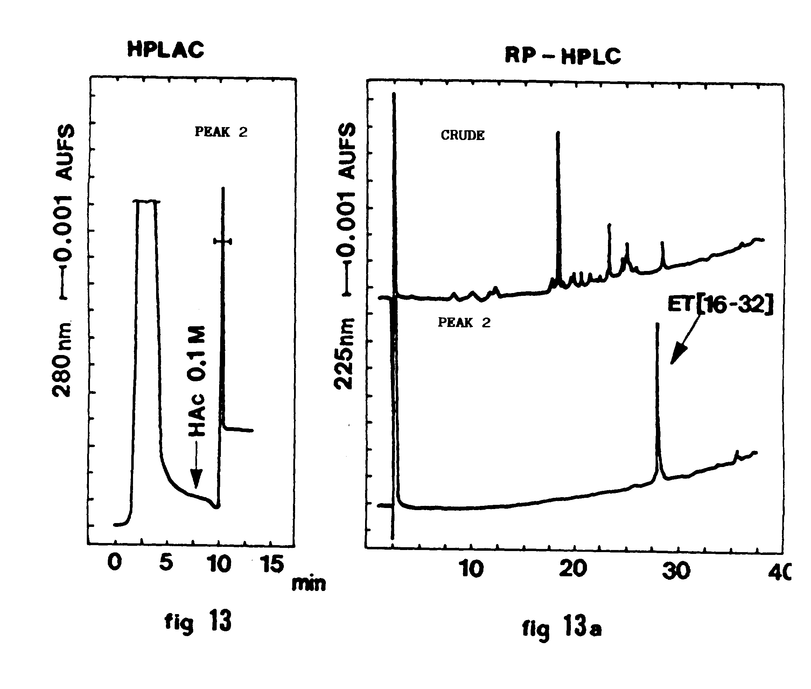

The two peptides were produced by chemical synthesis in solid phase with the Fmoc method, purified on RP-HPLC and their correct chemical nature was confirmed by means of amino acid analysis. Alike to the previous examples, the [CGET] peptide was immobilized upon on the support Eupergit [R] C30 n to prepare an affinity column. The total amount of immobilized [CGET] was of 500 μmoles determined by amino acid analysis of pondered amounts of derivatized support. In this case too, the elution of the peptide ET [16-32] from the column equilibrated with 0.1 M TRIS pH 7.0 can only be achieved by replacing the eluent with 0.1 M HAc (Figs 13 and 13a).

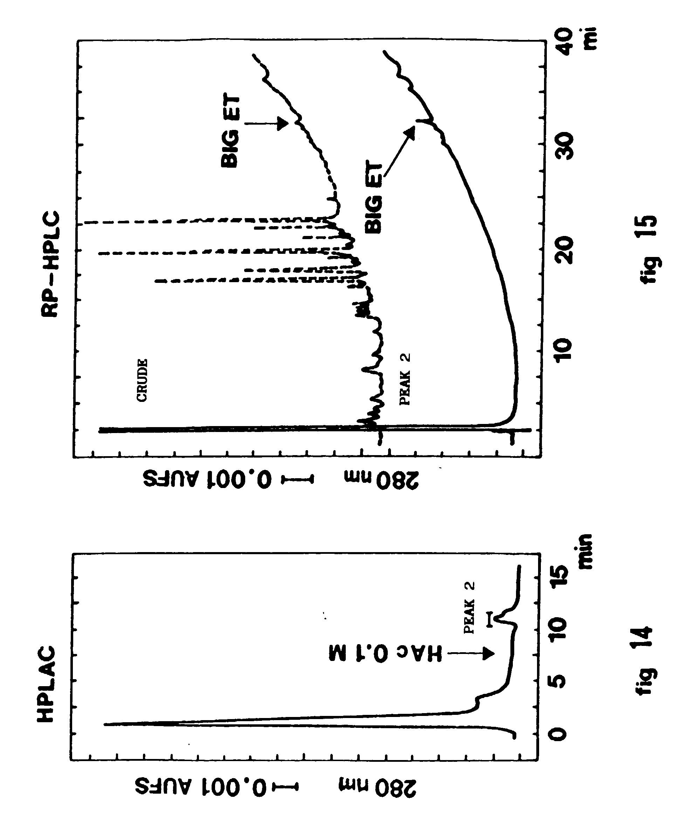

The affinity column showed also in this case a selectivity sufficient to enable the purification of ET [16-32] and of Big ET [1-38] from crude complex mixtures comprising the target peptides in concentration 1-2 μg/ml (Figs 14 and 15).

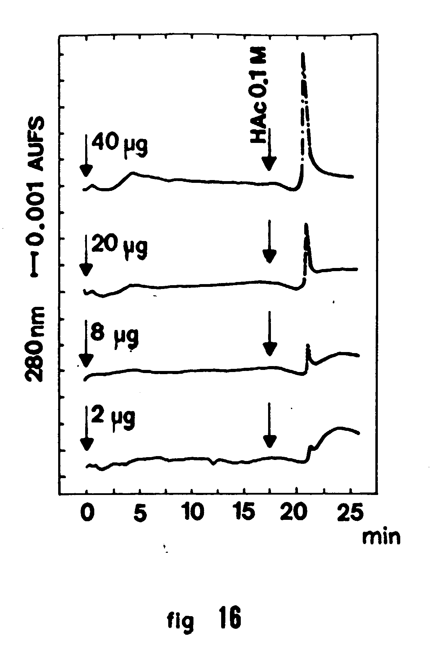

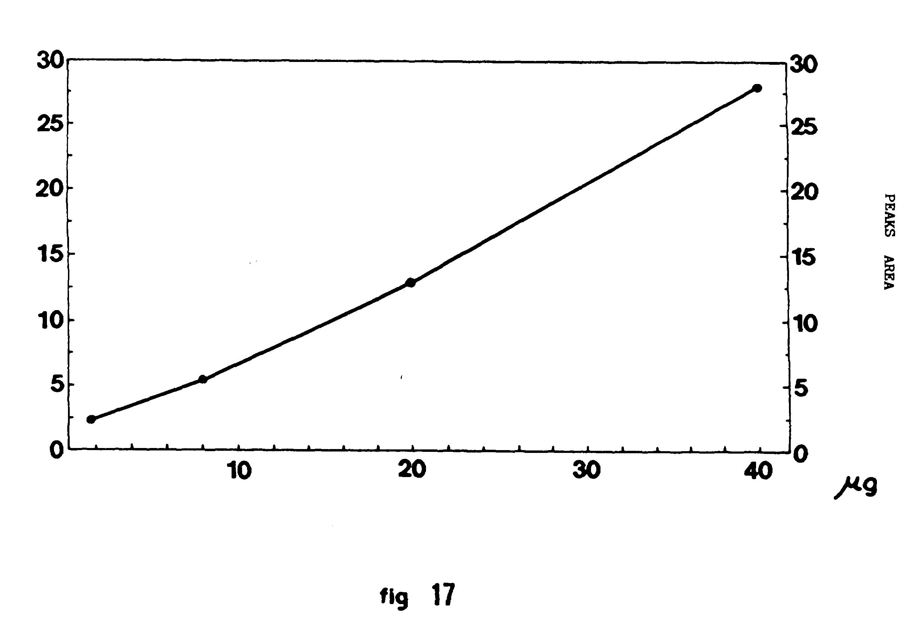

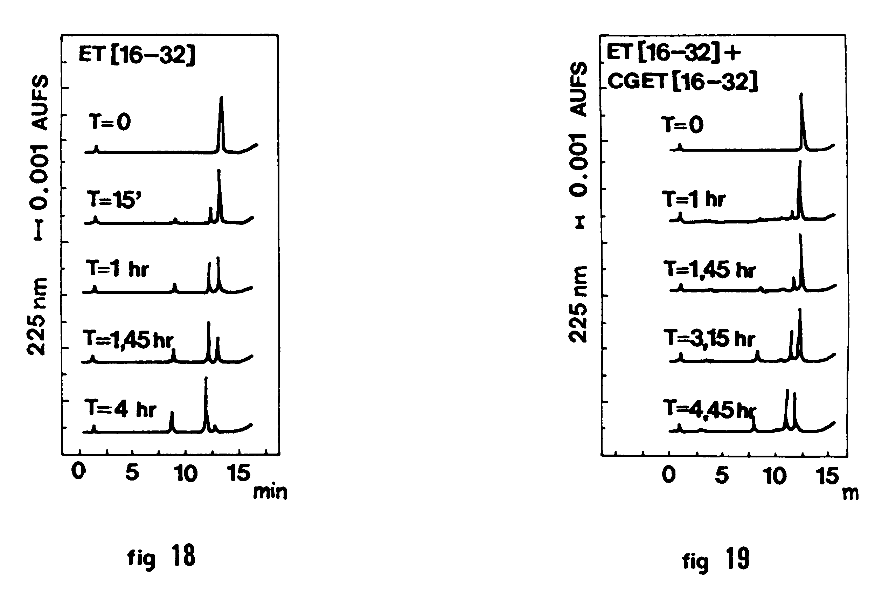

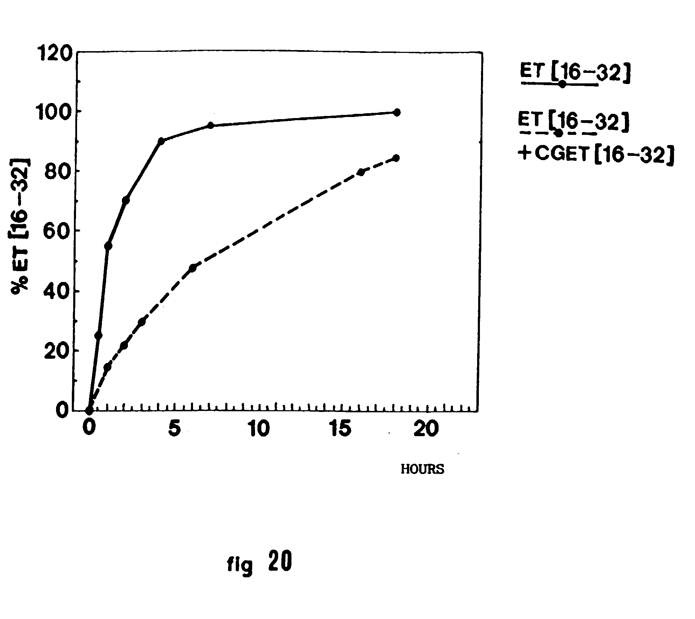

The so prepared affinity column resulted moreover to be of extreme usefulness for quantifying ET [1-38] within the range 2-40 μg (Figs 16 and 17). The effect of the complex formation between the complementary peptides [CGET] and ET [16-32] on the ET [16-32] degradation rate by the enzyme chymotrypsin provided further evidence of the binding. The degradation rate with both peptides in concentration 50 μg/ml appears to dramatically decrease as compared to the degradation rate of the peptide ET [16-32] alone. Hence the formation of the complex between the complementary pair promotes a protective function from proteolytic degradation. (Figs 18, 19 and 20).

## Claims

1. A process for the identification and synthesis of binding sites between two proteins and synthesis of peptides complementary to one target protein of said two proteins, wherein:

   a) the amino acid sequence of the two interacting proteins is determined;
   b) the hydropathic value for each element of the sequence of both proteins is determined by means of hydropatic values according to

TABLE 1:

| AminoAcid | | Value | Amino-Acid | | Value |
|---|---|---|---|---|---|
| Ala | (A) | 2.8 | Leu | (L) | 4.8 |
| Arg | (R) | -3.5 | Lys | (K) | -2.9 |
| Asn | (N) | -2.5 | Met | (M) | 2.9 |
| Asp | (D) | -2.5 | Phe | (F) | 3.8 |

TABLE 1: (continued)

| AminoAcid | | Value | Amino-Acid | | Value |
|---|---|---|---|---|---|
| Cys | (C) | 3.5 | Pro | (P) | -0.6 |
| Gln | (Q) | -2.5 | Ser | (S) | 0.2 |
| Glu | (E) | -2.5 | Thr | (T) | 0.3 |
| Gly | (G) | 0.6 | Trp | (W) | 0.1 |
| His | (H) | -2.2 | Tyr | (Y) | -0.3 |
| Ile | (I) | 5.5 | Val | (V) | 5.2 |

c) the hydropathic values for the two proteins are summed according to the formulas:

$$a_i = \Sigma\, h_k\, (P_k)/r \qquad b_i = \Sigma\, h_k\, (P_k)\, /r$$

where $i = (i+s)$ and $k$ $(i-s)$ and where $r$ is an integer defined as the run length ($r < n$) and $s$ is equal to $(r-1)/2$ per each residue $i$ of the two proteins;

d) the complementary hydropathic score for a given search length L is calculated according to the formula:

$$\theta = [\Sigma\, (a_i + b_i)^2 /L]^{1/2}$$

where L is lower than the total number of amino acids comprised in the proteins under consideration;

e) at least a pair of sequences of length L for the interacting couple, exhibiting the lowest score, are selected;

f) the found sequences are synthesized.

g) the amino acid sequence Pi of the target peptide or protein is determined:

$$Pi = P_1 - P_2 - P_3 - P_4 \ldots. P_n;$$

h) the hydropathic value h to be attributed to each residue of the sequence Pi (target) is deduced by means of the hydropathy scale

(Table 2): $h_1 - h_2 - h_3 - h_4 \ldots. h_n$;

TABLE 2

| Aminoacid | | Hydropathic value | Inverses |
|---|---|---|---|
| Ala | (A) | 1.8 | P,S,W,Y |
| Arg | (R) | - 4.5 | I,L,V |
| Asn | (N) | - 3.5 | C,I,L,F,V |
| Asp | (D) | - 3.5 | C,I,L,F,V |
| Cys | (C) | 2.5 | N,D,Q,E,H,P |
| Gln | (Q) | - 3.5 | C,I,L,F,V |
| Glu | (E) | - 3.5 | C,I,L,F,V |
| Gly | (G) | - 0.4 | G |
| His | (H) | - 3.2 | C,L,F,V |
| Ile | (I) | 4.5 | R,N,D,Q,E,K |
| Leu | (L) | 3.8 | R,N,D,Q,E,H,K |
| Lys | (K) | - 3.9 | I,L,V |
| Met | (M) | 1.9 | P,W,Y |
| Phe | (F) | 2.8 | N,D,Q,E,H |
| Pro | (P) | - 1.6 | A,C,M |
| Ser | (S) | - 0.8 | A |
| Thr | (T) | - 0.7 | A,G |
| Trp | (W) | - 0.9 | A,M |

TABLE 2   (continued)

| Aminoacid | | Hydropathic value | Inverses |
|---|---|---|---|
| Tyr | (Y) | - 1.3 | A,M |
| Val | (V) | 4.2 | R,N,D,Q,E,H,K. |

i) a set of complementary amino acids Q is attributed to each residue of the sequence P:

$$P_1 \rightarrow h_1 \rightarrow (Q_{1,1} - Q_{1,2} - Q_{1,3} .... Q_{1,m})$$
$$P_2 \rightarrow h_2 \rightarrow (Q_{2,1} - Q_{2,2} - Q_{2,3} .... Q_{2,m})$$
$$P_n \rightarrow h_n \rightarrow (Q_{n,1} - Q_{n,2} - Q_{n,3} .... Q_{n,m})$$

k) the hydropathic values of the target sequence are summed in order to obtain the average value of hydropathy flowing along the sequence according to the formula:

$$a_i = \Sigma_k \, h_r \, (P_k)/r$$

where $a_i$ is the average value of flowing hydropathy, **h** is the hydropathic value, P is the peptide sequence, **k** is the constant comprised within (i-s) and (i+s), **i** is higher than **s** and lower than or equal to (n-s), **s** is (r-1)/2, with **r** odd number equal to or lower than **n** and **n** is the number of amino acids comprised in the sequence under consideration;

l) the hydrophatic values of the series of the hydropathically complementary amino acids are summed in such a way to obtain the average values of flowing hydropathy for each member of the complementary series according to the formula:

$$b_{j,i} = \Sigma_k h'_{j,k}(P_{j,k})/r$$

where $b_{j,i}$ is the average value of the flowing hydropathy, k is comprised between (i-s) and (i+s), **i** is higher than s and lower than or equal to (n-s), **j** is comprised between 1 and the total number of sequences of the complementary series, **s** is (r-1)/2, **r** is an odd number of amino acids comprised in the complementary series;

m) the score of hydropathic complementarity θ is calculated and defined as:

$$\theta = [\Sigma(a_{i,k} + b_{j,i,k})^2/(n\text{-}2S)]J^{1/2}$$

where **k** is comprised between (s-1) and (s+1), **n** and **s** are defined as above;

n) the complementary sequence exhibiting the lowest complementarity score θ is selected and identified as that having high affinity and selectivity in recognizing the target sequence;

o) the complementary amino acid sequence identified by the lowest complementarity score is synthesized according to known techniques.

2. The process according to claim 1, wherein the target amino acid sequence is of natural origin and available, wherein:

a) the amino acid sequence Pi of the target peptide or protein of natural origin is determined:

$$Pi = P_1 - P_2 - P_3 - P_4 .... P_n;$$

b) the hydropathic value **h** to be attributed to each residue of the sequence Pi (target) is deduced by means of the hydropathy scale
(Table 2): $h_1 - h_2 - h_3 - h_4 .... h_n$;

TABLE 2

| Aminoacid | | Hydropathic value | Inverses |
|---|---|---|---|
| Ala | (A) | 1.8 | P,S,W,Y |

TABLE 2 (continued)

| Aminoacid | | Hydropathic value | Inverses |
|---|---|---|---|
| Arg | (R) | - 4.5 | I,L,V |
| Asn | (N) | - 3.5 | C,I,L,F,V |
| Asp | (D) | - 3.5 | C,I,L,F,V |
| Cys | (C) | 2.5 | N,D,Q,E,H,P |
| Gln | (Q) | - 3.5 | C,I,L,F,V |
| Glu | (E) | - 3.5 | C,I,L,F,V |
| Gly | (G) | - 0.4 | G |
| His | (H) | - 3.2 | C,L,F,V |
| Ile | (I) | 4.5 | R,N,D,Q,E,K |
| Leu | (L) | 3.8 | R,N,D,Q,E,H,K |
| Lys | (K) | - 3.9 | I,L,V |
| Met | (M) | 1.9 | P,W,Y |
| Phe | (F) | 2.8 | N,D,Q,E,H |
| Pro | (P) | - 1.6 | A,C,M |
| Ser | (S) | - 0.8 | A |
| Thr | (T) | - 0.7 | A,G |
| Trp | (W) | - 0.9 | A,M |
| Tyr | (Y) | - 1.3 | A,M |
| Val | (V) | 4.2 | R,N,D,Q,E,H,K. |

c) a set of complementary amino acids Q is attributed to each residue of the sequence P:

$$P_1 \rightarrow h_1 \rightarrow (Q_{1,1} - Q_{1,2} - Q_{1,3} .... Q_{1,m})$$
$$P_2 \rightarrow h_2 \rightarrow (Q_{2,1} - Q_{2,2} - Q_{2,3} .... Q_{2,m})$$
$$P_n \rightarrow h_n \rightarrow (Q_{n,1} - Q_{n,2} - Q_{n,3} .... Q_{n,m})$$

d) the hydropathic values of the target sequence are summed in order to obtain the average value of hydropathy flowing along the sequence according to the formula:

$$a_i = \Sigma_k \, h_r \, (P_k)/r$$

where $a_i$ is the average value of flowing hydropathy, **h** is the hydropathic value, P is the peptide sequence, **k** is the constant comprised within (i-s) and (i+s), **i** is higher than **s** and lower than or equal to (n-s), **s** is (r-1)/2, with **r** odd number equal to or lower than **n** and **n** is the number of amino acids comprised in the sequence under consideration;

e) the hydropathic values of the series of the hydropathically complementary amino acids are summed in such a way to obtain the average values of flowing hydropathy for each member of the complementary series according to the formula:

$$b_{j,i} = \Sigma_k h'_{j,k} \, (P_{j,k})/r$$

where $b_{j,i}$ is the average value of the flowing hydropathy, k is comprised between (i-s) and (i+s), **i** is higher than s and lower than or equal to (n-s), **j** is comprised between 1 and the total number of sequences of the complementary series, **s** is (r-1)/2, **r** is an odd number of amino acids comprised in the complementary series;

f) the score of hydropathic complementarity θ is calculated and defined as:

$$\theta = [\Sigma(a_{i,k} + b_{j,i,k})^2/(n-2S)]J^{1/2}$$

where **k** is comprised between (s-1) and (s+1), **n** and **s** are defined as above;

g) the complementary sequence exhibiting the lowest complementarity score θ is selected and identified as that having high affinity and selectivity in recognizing the target sequence;

h) the complementary amino acid sequence identified by the lowest complementarity score is synthesized according to known techniques.

3. The process as claimed in claim 1, wherein the average value of hydropathy flowing along the protein chain is determined calculating by sequential method the arithmetic mean of hydropathic values of each aminoacid along the chain in groups of 3,5,7,9,11 residues.

4. The process as claimed in claim 1 or 2, wherein the target peptides are selected among :

   - sequence 429-439 of the protein HIV-1 gp 120, having the structure $NH_2$-APPISGOIRSS-COOH;
   - site [205-216] of the human interleukin 1β [KQYPKKKMERKRF] ; and
   - site 16-32 of Big Endotheline [1-38].

5. The process as claimed in claim 1 or 2, wherein :

   - the complementary peptide to site [205-216] of the human interleukin 1β [KQYPKKKMERKRF] is HLLFPIIIAASL;
   - the complementary peptide to site 16-32 of Big Endotheline [1-38] is RKFLAGLRARRLKR.

6. The process as claimed in claim 1 or 2, wherein the interacting proteins are HIV-1 qp120 and CD4, and the found binding sequences are the complementary regions 429-439 of HIV-1 qp120 protein, having the structure $NH_2$-APPISGOIRSS-COOH, and the site 61-51 of the CD4 receptor, having the structure $NH_2$-GPSKLNDRADS-COOH.

7. A peptide selected between $NH_2$-APPISGOIRSS-COOH and $NH_2$-GPSKLNDRADS-COOH.

8. A peptide selected between HLLFPIIIAASL and RKFLAGLRARRLKR.

9. The peptides according to claim 7 or 8, synthesized according to the process of claim 1.

10. Affinity column for purifying proteins and peptides consisting of the peptides claimed in claims 7, 8 or 9, binded on suitable solid support.

**Patentansprüche**

1. Verfahren zur Identifizierung und Synthese von Bindungsstellen zwischen zwei Proteinen und Synthese der zu einem Zielprotein der zwei Proteine komplementären Peptide, wobei:

   a) die Aminosäuresequenz der zwei interagierenden Proteine bestimmt wird;

   b) der Hydrophobizitätswert für jedes Element der Sequenz der beiden Proteine mit Hilfe der Hydrophobizitätswerte gemäß

Tabelle 1

| Aminosäure | | Wert | Aminosäure | | Wert |
|---|---|---|---|---|---|
| Ala | (A) | 2,8 | Leu | (L) | 4,8 |
| Arg | (R) | -3,5 | Lys | (K) | -2,9 |
| Asn | (N) | -2,5 | Met | (M) | 2,9 |
| Asp | (D) | -2,5 | Phe | (F) | 3,8 |
| Cys | (C) | 3,5 | Pro | (P) | -0,6 |
| Gln | (Q) | -2,5 | Ser | (S) | 0,2 |
| Glu | (E) | -2,5 | Thr | (T) | 0,3 |
| Gly | (G) | 0,6 | Trp | (W) | 0,1 |
| His | (H) | -2,2 | Tyr | (Y) | -0,3 |
| Ile | (I) | 5,5 | Val | (V) | 5,2 |

bestimmt wird.

c) die Hydrophobizitätswerte für die zwei Proteine gemäß den Formeln

$$a_i = \Sigma \, h_k \, (P_k)/r \qquad b_i = \Sigma \, h_k \, (P_k)/r$$

zusammengezählt werden, worin i = (i+s) und k = (i-s) und r eine ganze Zahl, definiert als Lauflänge (r < n) ist und s ist gleich (r-1)/2 für jeden Rest i der beiden Proteine;

d) der komplementäre hydrophobe Wert für eine gegebene Suchlänge L gemäß der folgenden Formel

$$\theta = [\Sigma \, (a_i + b_i)^2 \, /L]^{1/2}$$

berechnet wird, wobei L kleiner als die Gesamtzahl der enthaltenen Aminosäuren der zu betrachteten Proteine ist;

e) wenigstens ein Sequenzpaar der Länge L, das die niedrigste Punktzahl aufweist, als interagierendes Paar ausgewählt wird;

f) die gefundenen Sequenzen synthetisiert werden;

g) die Aminosäuresequenz Pi des Zielpeptids oder -proteins bestimmt wird:

$$Pi = P_1 \, \text{-}P_2 \, \text{-}P_3 \, \text{-}P_4 \, ....P_n;$$

h) der Hydrophobizitätswert h, der jedem Rest der Sequenz Pi (Ziel) zugeordnet wird, durch die Hydropathieskala hergeleitet wird (Tabelle 2): $h_1$ -$h_2$ -$h_3$ -$h_4$ ....$h_n$;

Tabelle 2

| Aminosäure | | Hydrophobizitätswert | Inverse |
|---|---|---|---|
| Ala | (A) | 1,8 | P,S,W,Y |
| Arg | (R) | -4,5 | I,L,V |
| Asn | (N) | -3,5 | C,I,L,F,V |
| Asp | (D) | -3,5 | C,I,L,F,V |
| Cys | (C) | 2,5 | N,D,Q,E,H,P |
| Gln | (Q) | -3,5 | C,I,L,F,V |
| Glu | (E) | -3,5 | C,I,L,F,V |
| Gly | (G) | -0,4 | G |
| His | (H) | -3,2 | C,L,F,V |
| Ile | (I) | 4,5 | R,N,D,Q,E,K |
| Leu | (L) | 3,8 | R,N,D,Q,E,H,K |
| Lys | (K) | -3,9 | I,L,V |
| Met | (M) | 1,9 | P,W,Y |
| Phe | (F) | 2,8 | N,D,Q,E,H |
| Pro | (P) | -1,6 | A,C,M |
| Ser | (S) | -0,8 | A |
| Thr | (T) | -0,7 | A,G |
| Trp | (W) | -0,9 | A,M |
| Tyr | (Y) | -1,3 | A,M |
| Val | (V) | 4,2 | R,N,D,Q,E,H,K |

i) ein Satz von komplementären Aminosäuren Q jedem Rest der Sequenz P zugeordnet wird:

$P_1 \rightarrow h_1 \rightarrow (Q_{1,1} \, \text{-}Q_{1,2}, \, \text{-}Q_{1,3} \, ....Q_{1,m})$
$P_2 \rightarrow h_2 \rightarrow (Q_{2,1} \, \text{-}Q_{2,2}, \, \text{-}Q_{2,3} \, ....Q_{2,m})$

$$P_n \rightarrow h_n \rightarrow (Q_{n,1} \text{-}Q_{n,2}, \text{-}Q_{n,3} \dots Q_{n,m});$$

k) die Hydrophobizitätswerte der Zielsequenz zusammengezählt werden, um den Durchschnittswert der Hydropathie entlang der Sequenz gemäß der Formel

$$a_i = \Sigma_k \, h_r \, (P_k)/r$$

zu erhalten, wobei $a_i$ der Durchschnittswert der laufenden Hydropathie ist, h ist der Hydrophobizitätswert, P ist die Peptidsequenz, k ist die Konstante die zwischen (i-s) und (i+s) liegt, i ist größer als s und niedriger oder gleich (n-s), s ist (r-1)/2, wobei r eine ungerade Zahl gleich oder kleiner n ist und n die Zahl der Aminosäuren der in Betracht kommenden Sequenz ist;

l) die Hydrophobizitätswerte der Serien der hydrophatischen komplementären Aminosäuren zusammengezählt werden, so daß Durchschnittswerte für die laufende Hydropathie für jedes Mitglied der komplementären Serien gemäß der Formel

$$b_{j,i} = \Sigma_k h'_{j,k}(P_{j,k})/r$$

erhalten wird, wobei $b_{j,i}$ der Durchschnittswert der laufenden Hydropathie ist, k liegt zwischen (i-s) und (i+s), i ist größer als s und kleiner oder gleich (n-s), j liegt zwischen 1 und der Gesamtzahl der Sequenzen der komplementären Serien, s ist (r-1)/2, r ist eine ungerade Zahl von Aminosäuren enthalten in den komplementären Serien;

m) der Wert der Hydrophobizitätskomplementarität $\theta$ berechnet wird und definiert ist als:

$$\theta = [\Sigma(a_{i,k} + b_{j,i,k})^2/(n\text{-}2S)]J^{1/2}$$

wobei k zwischen (s-1) und (s+1) liegt, n und s sind wie oben definiert;

n) die komplementäre Sequenz, die den niedrigsten Komplementaritätswert $\theta$ aufweist, als die ausgewählt und identifiziert wird, die eine starke Affinität und Selektivität bei der Erkennung der Zielsequenz hat;

o) die durch den niedrigsten Komplementaritätswert identifizierte komplementäre Aminosäuresequenz gemäß bekannten Techniken synthetisiert wird.

**2.** Verfahren gemäß Anspruch 1, worin die Ziel-Aminosäuresequenz natürlichen Ursprungs und von dort erhältlich ist, wobei:

a) die Aminosäuresequenz Pi des Zielpeptids oder -proteins natürlichen Ursprungs bestimmt wird:

$$Pi = P_1 \text{-}P_2 \text{-}P_3 \text{-}P_4 \dots P_n;$$

b) der Hydrophobizitätswert h, der jedem Rest der Sequenz Pi (Ziel) zugeordnet wird, sich herleiten läßt durch die Hydropathieskala (Tabelle 2): $h_1 \text{-}h_2 \text{-}h_3 \text{-}h_4 \dots h_n;$

Tabelle 2

| Aminosäure | | Hydrophobizitätswert | Inverse |
|---|---|---|---|
| Ala | (A) | 1,8 | P,S,W,Y |
| Arg | (R) | -4,5 | I,L,V |
| Asn | (N) | -3,5 | C,I,L,F,V |
| Asp | (D) | -3,5 | C,I,L,F,V |
| Cys | (C) | 2,5 | N,D,Q,E,H,P |

Tabelle 2   (fortgesetzt)

| Aminosäure | | Hydrophobizitätswert | Inverse |
|---|---|---|---|
| Gln | (Q) | -3,5 | C,I,L,F,V |
| Glu | (E) | -3,5 | C,I,L,F,V |
| Gly | (G) | -0,4 | G |
| His | (H) | -3,2 | C,L,F,V |
| Ile | (I) | 4,5 | R,N,D,Q,E,K |
| Leu | (L) | 3,8 | R,N,D,Q,E,H,K |
| Lys | (K) | -3,9 | I,L,V |
| Met | (M) | 1,9 | P,W,Y |
| Phe | (F) | 2,8 | N,D,Q,E,H |
| Pro | (P) | -1,6 | A,C,M |
| Ser | (S) | -0,8 | A |
| Thr | (T) | -0,7 | A,G |
| Trp | (W) | -0,9 | A,M |
| Tyr | (Y) | -1,3 | A,M |
| Val | (V) | 4,2 | R,N,D,Q,E,H,K |

c) ein Satz komplementärer Aminosäuren Q jedem Rest der Sequenz P zugeordnet wird:

$$P_1 \rightarrow h_1 \rightarrow (Q_{1,1} -Q_{1,2}, -Q_{1,3} .... Q_{1,m})$$
$$P_2 \rightarrow h_2 \rightarrow (Q_{2,1} -Q_{2,2}, -Q_{2,3} .... Q_{2,m})$$
$$P_n \rightarrow h_n \rightarrow (Q_{n,1} -Q_{n,2}, -Q_{n,3} .... Q_{n,m});$$

d) die Hydrophobizitätswerte der Zielsequenz zusammengezählt werden, um den Durchschnittswert der Hydropathie entlang der Sequenz gemäß der Formel

$$a_i = \Sigma_k \, h_r \, (P_k)/r$$

zu erhalten, wobei $a_i$ der Durchschnittswert der laufenden Hydropathie ist, h ist der Hydrophobizitätswert, P ist die Peptidsequenz, k ist die Konstante, die innnerhalb (i-s) und (i+s) liegt, i ist größer als s und niedriger oder gleich (n-s), s ist (r-1)/2, wobei r eine ungerade Zahl gleich oder kleiner als n ist, und n die Anzahl der in der zu Betracht ziehenden Sequenz enthaltenen Aminosäuren ist;

e) die Hydrophobizitätswerte der Serien an hydrophatischen komplementären Aminosäuren zusammengezählt werden, so daß Durchschnittswerte der laufenden Hydropathie für jedes Mitglied der komplementären Serien gemäß der Formel

$$b_{j,i} = \Sigma_k h'_{j,k}(P_{j,k})/r$$

erhalten werden, wobei $b_{j,i}$ der Durchschnittswert der laufenden Hydropathie ist, k liegt zwischen (i-s) und (i+s), i ist größer als s und kleiner oder gleich (n-s), j liegt zwischen 1 und der Gesamtzahl der Sequenzen der komplementären Serien, s ist (r-1)/2, r ist eine ungerade Zahl von Aminosäuren enthalten in den komplementären Serien;

f) der Wert der Hydrophobizitätskomplementarität θ berechnet wird und definiert ist als:

$$\theta = [\Sigma(a_{i,k} + b_{j,i,k})^2/(n-2S)]^{1/2}$$

wobei k zwischen (s-1) und (s+1) liegt, n und s sind wie oben definiert;

g) die komplementäre Sequenz, die den niedrigsten Komplementaritätswert θ aufweist, als die ausgewählt und identifiziert wird, die eine starke Affinität und Selektivität bei der Erkennung der Zielsequenz hat;

h) die durch den niedrigsten Komplementaritätswert identifizierte komplementäre Aminosäuresequenz gemäß bekannten Techniken synthetisiert wird.

3.  Verfahren gemäß Anspruch 1, wobei der Durchschnittswert der Hydropathie entlang der Proteinkette bestimmt wird durch Berechnen mit Hilfe von sequentiellen Verfahren des arithmetischen Mittels der Hydrophobizitätswerte jeder Aminosäure entlang der Kette in Gruppen von 3,5,7,9,11 Resten.

4.  Verfahren gemäß Anspruch 1 oder 2, wobei die Zielpeptide ausgewählt werden unter:

    -  Sequenz 429-439 des HIV-1gp120 Proteins mit der Struktur $NH_2$-APPISGOIRSS-COOH;
    -  Stelle [205-216] des humanen Interleukins 1β [KQYPKKKMERKRF]; und
    -  Stelle 16-32 vom großen Endothelin [1-38].

5.  Verfahren gemäß Anspruch 1 oder 2, worin:

    -  das komplementäre Peptid zu der Stelle [205-216] des humanen Interleukins 1β [KQYPKKKMERKRF] HLLFPIIIAASL ist;
    -  das komplementäre Peptid zu der Stelle 16-32 des großen Endothelins [1-38] RKFLAGLRARRLKR ist.

6.  Verfahren gemäß Anspruch 1 oder 2, worin die interagierenden Proteine HIV-1 gp120 und CD4 sind und die gefundene Bindungssequenz die komplementären Bereiche 429-439 des HIV-1 gp120 Proteins mit der Struktur $NH_2$-APPISGOIRSS-COOH der Stelle 61-51 des CD4 Rezeptors mit der Struktur $NH_2$-GPSKLNDRADS-COOH sind.

7.  Peptid ausgewählt aus $NH_2$-APPISGOIRSS-COOH und $NH_2$-GPSKLNDRADS-COOH.

8.  Peptid ausgewählt aus HLLFPIIIAASL und RKFLAGLRARRLKR.

9.  Peptide gemäß Anspruch 7 oder 8, die gemäß den Verfahren nach Anspruch 1 synthetisiert wurden.

10.  Affinitätssäule zur Aufreinigung von Proteinen und Peptiden bestehend aus Peptiden gemäß Anspruch 7, 8 oder 9, die an einen geeigneten festen Träger gebunden sind.

**Revendications**

1.  Procédé pour l'identification et la synthèse de sites de liaison entre deux protéines et la synthèse de peptides complémentaires d'une protéine cible desdites deux protéines, dans lequel :

    a) la séquence d'acides aminés des deux protéines qui interagissent est déterminée ;
    b) la valeur hydropathique pour chaque élément de la séquence des deux protéines est déterminée au moyen des valeurs hydropathiques suivant le :

TABLEAU 1

| Acide aminé | | Valeur | Acide aminé | | Valeur |
|---|---|---|---|---|---|
| Ala | (A) | 2,8 | Leu | (L) | 4,8 |
| Arg | (R) | -3,5 | Lys | (K) | -2,9 |
| Asn | (N) | -2,5 | Met | (M) | 2,9 |
| Asp | (D) | -2,5 | Phe | (F) | 3,8 |
| Cys | (C) | 3,5 | Pro | (P) | -0,6 |
| Gln | (Q) | -2,5 | Ser | (S) | 0,2 |
| Glu | (E) | -2,5 | Thr | (T) | 0,3 |
| Gly | (G) | 0,6 | Trp | (W) | 0,1 |
| His | (H) | -2,2 | Tyr | (Y) | -0,3 |
| Ile | (I) | 5,5 | Val | (V) | 5,2 |

c) les valeurs hydropathiques pour les deux protéines sont sommées selon les formules :

$$a_i = \Sigma \, h_k \, (P_k)/r$$

$$b_i = \Sigma \, h_k \, (P_k)/r$$

où i = (i+s) et k (i-s) et où r est un entier défini comme étant la longueur de la course (r<n) et s est égal à (r-1) /2 pour chaque résidu des deux protéines ;

d) la note hydropathique complémentaire pour une longueur L de recherche donnée est calculée selon la formule :

$$\theta = [\Sigma \, (a_i + b_i)^2 / L]^{1/2}$$

où L est inférieur au nombre total d'acides aminés compris dans les protéines en question ;

e) au moins une paire de séquences de longueur L pour le couple qui interagit, présentant la note la plus faible, est choisie ;

f) les séquences trouvées sont synthétisées ;

g) la séquence Pi d'acides aminés du peptide ou de la protéine cible est déterminée :

$$P_i = P_1 - P_2 - P_3 - P_4 \, .... \, P_n \, ;$$

h) la valeur hydropathique h à attribuer à chaque résidu de la séquence Pi (cible) est déduite au moyen de l'échelle d'hydropathie (Tableau 2) : $h_1 - h_2 - h_3 - h_4 \, ... h_n$ ;

TABLEAU 2

| Acide aminé | | Valeur hydropathique | Inverses |
|---|---|---|---|
| Ala | (A) | 1,8 | P, S, W, Y |
| Arg | (R) | -4,5 | I, L, V |
| Asn | (N) | -3,5 | C, I, L, F, V |
| Asp | (D) | -3,5 | C, I, L, F, V |
| Cys | (C) | 2,5 | N, D, Q, E, H, P |
| Gln | (Q) | -3,5 | C, I, L, F, V |
| Glu | (E) | -3,5 | C, I, L, F, V |
| Gly | (G) | -0,4 | G |
| His | (H) | -3,2 | C, L, F, V |
| Ile | (I) | 4,5 | R, N, D, Q, E, K |
| Leu | (L) | 3,8 | R, N, D, Q, E, H, K |
| Lys | (K) | -3,9 | I, L, V |
| Met | (M) | 1,9 | P, W, Y |
| Phe | (F) | 2,8 | N, D, Q, E, H |
| Pro | (P) | -1,6 | A, C, M |
| Ser | (S) | -0,8 | A |
| Thr | (T) | -0,7 | A, G |
| Trp | (W) | -0,9 | A, M |
| Tyr | (Y) | -1,3 | A, M |
| Val | (V) | 4,2 | R, N, D, Q, E, H, K |

i) un ensemble d'acides aminés complémentaires Q est attribué à chaque résidu de la séquence P :

$$P_1 \rightarrow h_1 \rightarrow (Q_{1,1} - Q_{1,2} - Q_{1,3} \, .... \, Q_{1,m})$$
$$P_2 \rightarrow h_2 \rightarrow (Q_{2,1} - Q_{2,2} - Q_{2,3} \, .... \, Q_{2,m})$$
$$P_n \rightarrow h_n \rightarrow (Q_{n,1} - Q_{n,2} - Q_{n,3} \, .... \, Q_{n,m})$$

k) les valeurs hydropathiques de la séquence cible sont sommées pour obtenir la valeur moyenne mobile d'hydropathie le long de la séquence selon la formule :

$$a_i = \Sigma_k\, h_r\, (P_k)\, /\, r$$

où $a_i$ est la valeur moyenne mobile de l'hydropathie, h est la valeur hydropathique, P est la séquence du peptide, k est la constante comprise entre (i-s) et (i+s), i est supérieur à s et inférieur ou égal à (n-s), s est (r-1)/2, avec r nombre impair égal ou inférieur à n et n est le nombre d'acides aminés compris dans la séquence en question ;
l) les valeurs hydropathiques des séries des acides aminés hydropathiquement complémentaires sont sommées de façon à obtenir les valeurs moyennes mobiles d'hydropathie pour chaque membre des séries complémentaires suivant la formule :

$$b_{j,i} = \Sigma_k\, h'_{j,k}\, (P_{j,k})/r$$

où $b_{j,i}$ est la valeur moyenne mobile de l'hydropathie, k est compris entre (i-s) et (i+s), i est supérieur à s et inférieur ou égal à (n-s), j est compris entre 1 et le nombre total de séquences des séries complémentaires, s est (r-1) /2, r est un nombre impair d'acides aminés compris dans les séries complémentaires ;
m) la note de complémentarité hydropathique θ est calculée et définie par :

$$\Theta = [\Sigma(a_{i,k} + b_{j,i,k})^2 / (n- 2S)]J^{1/2}$$

où k est compris entre (s-1) et (s+1), n et s sont définis plus haut ;
n) la séquence complémentaire présentant la note de complémentarité Θ la plus faible est choisie et identifiée en tant que celle qui a de fortes affinité et sélectivité dans la reconnaissance de la séquence cible ;
o) la séquence d'acides aminés complémentaire identifiée par la plus faible note de complémentarité est synthétisée selon des techniques connues.

2. Procédé suivant la revendication 1, dans lequel la séquence d'acides aminés cible est d'origine naturelle et disponible, dans lequel :

a) la séquence $P_i$ d'acides aminés du peptide ou de la protéine cible d'origine naturelle est déterminée :

$$P_i = P_1 - P_2 - P_3 - P_4 .... P_n\, ;$$

b) la valeur hydropathique h à attribuer à chaque résidu de la séquence $P_i$ (cible) est déduite au moyen de l'échelle d'hydropathie (Tableau 2) : $h_1 - h_2 - h_3 - h_4 ... h_n$ ;

TABLEAU 2

| Acide aminé | | Valeur hydropathique | Inverses |
|---|---|---|---|
| Ala | (A) | 1,8 | P, S, W, Y |
| Arg | (R) | -4,5 | I, L, V |
| Asn | (N) | -3,5 | C, I, L, F, V |
| Asp | (D) | -3,5 | C, I, L, F, V |
| Cys | (C) | 2,5 | N, D, Q, E, H, P |
| Gln | (Q) | -3,5 | C, I, L, F, V |
| Glu | (E) | -3,5 | C, I, L, F, V |
| Gly | (G) | -0,4 | G |
| His | (H) | -3,2 | C, L, F, V |
| Ile | (I) | 4,5 | R, N, D, Q, E, K |
| Leu | (L) | 3,8 | R, N, D, Q, E, H, K |
| Lys | (K) | -3,9 | I, L, V |
| Met | (M) | 1,9 | P, W, Y |
| Phe | (F) | 2,8 | N, D, Q, E, H |

TABLEAU 2   (suite)

| Acide aminé | | Valeur hydropathique | Inverses |
|---|---|---|---|
| Pro | (P) | -1,6 | A, C, M |
| Ser | (S) | -0,8 | A |
| Thr | (T) | -0,7 | A, G |
| Trp | (W) | -0,9 | A, M |
| Tyr | (Y) | -1,3 | A, M |
| Val | (V) | 4,2 | R, N, D, Q, E, H, K |

c) un ensemble d'acides aminés complémentaires Q est attribué à chaque résidu de la séquence P :

$$P_1 \rightarrow h_1 \rightarrow (Q_{1,1} - Q_{1,2} - Q_{1,3} .... Q_{1,m})$$
$$P_2 \rightarrow h_2 \rightarrow (Q_{2,1} - Q_{2,2} - Q_{2,3} .... Q_{2,m})$$
$$P_n \rightarrow h_n \rightarrow (Q_{n,1} - Q_{n,2} - Q_{n,3} .... Q_{n,m})$$

d) les valeurs hydropathiques de la séquence cible sont sommées pour obtenir la valeur moyenne mobile d'hydropathie le long de la séquence selon la formule :

$$a_i = \Sigma_k\, h_r\, (P_k)\, /\, r$$

où $a_i$ est la valeur moyenne mobile de l'hydropathie, h est la valeur hydropathique, P est la séquence du peptide, k est la constante comprise entre (i-s) et (i+s), i est supérieur à s et inférieur ou égal à (n-s), s est (r-1)/2, avec r nombre impair égal ou inférieur à n et n est le nombre d'acides aminés compris dans la séquence en question ;

e) les valeurs hydropathiques des séries des acides aminés hydropathiquement complémentaires sont sommées de façon à obtenir les valeurs moyennes mobiles d'hydropathie pour chaque membre des séries complémentaires suivant la formule :

$$b_{j,i} = \Sigma_k\, h'_{j,k}\, (P_{j,k})/r$$

où $b_{j,i}$ est la valeur moyenne mobile de l'hydropathie, k est compris entre (i-s) et (i+s), i est supérieur à s et inférieur ou égal à (n-s), j est compris entre 1 et le nombre total de séquences des séries complémentaires, s est (r-1) /2, r est un nombre impair d'acides aminés compris dans les séries complémentaires ;

f) la note de complémentarité hydropathique $\Theta$ est calculée et définie par :

$$\Theta = [\Sigma\, (a_{i,k} + b_{j,i,k})^2/(n\text{-}2S)]J^{1/2}$$

où k est compris entre (s-1) et (s+1), n et s sont définis plus haut ;

g) la séquence complémentaire présentant la note de complémentarité $\Theta$ la plus faible est choisie et identifiée en tant que celle qui a de fortes affinité et sélectivité dans la reconnaissance de la séquence cible ;

h) la séquence d'acides aminés complémentaire identifiée par la plus faible note de complémentarité est synthétisée selon des techniques connues.

3. Procédé suivant la revendication 1, dans lequel la valeur moyenne mobile de l'hydropathie le long de la chaîne de la protéine est déterminée par calcul selon une méthode séquentielle de la moyenne arithmétique des valeurs hydropathiques de chaque acide aminé le long de la chaîne en groupes de 3, 5, 7, 9, 11 résidus.

4. Procédé suivant les revendications 1 ou 2, dans lequel les peptides cibles sont choisis parmi :

-   la séquence 429-439 de la protéine HIV-1 gp 120, ayant la structure $NH_2$-APPISGOIRSS-COOH ;
-   le site [205-216] de l'interleukine 1β humaine [KQYPKKKMERKRF] ; et
-   le site 16-32 de Big Endotheline [1-38].

5. Procédé suivant les revendications 1 ou 2, dans lequel :

- le peptide complémentaire du site [205-216] de l'interleukine 1β humaine [KQYPKKKMERKRF] est HLLFPIIIAASL ; et
- le peptide complémentaire du site 16-32 de Big Endotheline [1-38] est RKFLAGLRARRLKR.

6.  Procédé suivant les revendications 1 ou 2, dans lequel les protéines qui interagissent sont HIV-1 gp120 et CD4, et les séquences de liaison trouvées sont les régions complémentaires 429-439 de la protéine HIV-1 gp120 qui a la structure $NH_2$-APPISGOIRSS-COOH, et le site 61-51 du récepteur CD4 qui a la structure $NH_2$-GPSKLNDRADS-COOH.

7.  Peptide choisi parmi $NH_2$-APPISGOIRSS-COOH et $NH_2$-GPSKLNDRADS-COOH.

8.  Peptide choisi parmi HLLFPIIIAASL et RKFLAGLRARRLKR.

9.  Peptides suivant les revendications 7 ou 8, synthétisés suivant le procédé de la revendication 1.

10. Colonne d'affinité pour la purification de protéines et de peptides consistant en peptides suivant les revendications 7, 8 ou 9, liés à un support solide convenables.

fig 1

fig 2

EP 0 411 503 B1

fig 3

27

fig 4

fig 5

fig 6

EP 0 411 503 B1

fig 7

31

# HPLAC

# RP – HPLC

fig 8

fig 9

EP 0 411 503 B1

fig 10a — HPLAC — PEAK 2 — HAc 0.1 M — 225 nm ⊢—⊣ 0.001 AUFS

fig 10b — RP-HPLC — PEAK 2 — CRUDE — 225 nm ⊢—⊣ 0.001 AUFS

fig 10c — HPEC — PEAK 2 — CRUDE

33

fig 11

fig 12

ET [16−32]
HLDIIWVNTPEHIVF

CGET [16−32]
RKFLAGLRARRLKI

EP 0 411 503 B1

HPLAC

280 nm ⟶ 0.001 AUFS

PEAK 2

HAc 0.1 M

fig 13

RP–HPLC

225 nm ⟶ 0.001 AUFS

CRUDE

PEAK 2

ET[16-32]

fig 13a

EP 0 411 503 B1

**HPLAC**

280 nm $\vdash$ 0.001 AUFS

HAc 0.1 M

PEAK 2

0    5    10    15    min

**fig 14**

**RP-HPLC**

280 nm $\vdash$ 0.001 AUFS

CRUDE

BIG ET

PEAK 2

BIG ET

10    20    30    40    mi

**fig 15**

EP 0 411 503 B1

fig 16

PEAKS AREA

µg

fig 17

fig 19

fig 18

fig 20